# EUROPÄISCHE PATENTSCHRIFT

(11) **EP 4 105 311 B1**
(45) Veröffentlichungstag und Bekanntmachung des Hinweises auf die Patenterteilung: **01.11.2023**
(21) Anmeldenummer: 21179865.7
(22) Anmeldetag: 16.06.2021
(51) Int. Cl.: C12M 1/00

(54) **VORRICHTUNGSANORDNUNG UND VERFAHREN ZUR ABTRENNUNG VON ZELLEN AUS EINEM KULTURMEDIUM IN EINEM BIOPROZESS**
DEVICE AND METHOD FOR SEPARATING CELLS FROM A CULTURE MEDIUM IN A BIOPROCESS
AGENCEMENT DE DISPOSITIFS ET PROCÉDÉ DE SÉPARATION DES CELLULES D'UN MILIEU DE CULTURE DANS UN PROCESSUS BIOLOGIQUE

(43) Veröffentlichungstag der Anmeldung: 21.12.2022
(73) Patentinhaber: Sartorius Stedim Biotech GmbH, 37079 Göttingen (DE)
(72) Erfinder: DIEL, Bernhard, 37079 Göttingen (DE)
(74) Vertreter: Novagraaf International SA

(56) Entgegenhaltungen:
- EP-A2- 1 775 000
- WO-A1-2018/022661
- DE-T2- 69 432 563
- DE-T2- 69 526 889
- US-B1- 6 544 424

## Beschreibung

Die Erfindung betrifft eine Vorrichtungsanordnung und ein Verfahren zur Abtrennung von Zellen aus einem Kulturmedium in einem Bioprozess, insbesondere in einem biopharmazeutischen Prozess.

In der biopharmazeutischen Industrie ist ein deutlicher Trend in Richtung steigender Effizienz und Intensivierung der Kultivierungsprozesse zu beobachten. Sich ständig verbessernde Technologien und optimierte Verfahren führen zu immer kleineren Fermentationsvolumina bei gleichzeitig immer höheren Zell- und Proteinkonzentrationen, welche in den weiteren Prozessschritten zur Klärung und Isolation der eigentlichen Zielsubstanz verarbeitet werden müssen.

Daher kommt gerade dem ersten Klärungsschritt der Zellabtrennung, direkt nach der Kultivierung, eine immer größere Bedeutung zu, denn hohe Wirkstoffkonzentrationen gehen in der Regel auch mit hohen Zellzahlen und damit mit größeren Biofeuchtmassen einher. Die konventionellen Methoden zur Zellabtrennung, wie Zentrifugation und Tiefenfiltration sowie vereinzelt auch Querstromfiltration (Tangential-Flow-Filtration, TFF), werden hier also vor immer weiter wachsende Herausforderungen gestellt und stoßen dabei häufig an technische Grenzen.

Daneben ist in der biopharmazeutischen Industrie ein stetig steigendes Interesse an Einweg-Prozesslösungen (single use) zu beobachten, mit dem Ziel, kosten- und zeitintensive Validierungsprozesse von Edelstahlsystemen - bei allen Zwischenstufen auf dem Weg der Entwicklung eines neuen Medikamentes - zu optimieren bzw. zu beschleunigen. Die zunehmende Akzeptanz von Einweg-Technologien, die sich über den gesamten Herstellungsprozess von Biopharmazeutika erstreckt, führt dazu, dass ständig neue und innovative Technologien und Techniken gesucht werden, die diesen Herausforderungen Rechnung tragen. Auch bei dem am Anfang der Prozesskette stehenden Fermentationsprozess ist die Verwendung von Einweg-Bioreaktoren im Vormarsch und heute bereits sehr weit verbreitet. Der der Fermentation folgende Schritt der Zellabtrennung wird in Einweg-Produktionsanlagen in der Regel mit zwei oder mehreren Tiefenfiltrationsschritten bewerkstellig. Traditionell ist diese Filtrationstechnologie jedoch in Bezug auf die Konzentration der abzutrennenden Zellmassen limitiert, da einer schnellen Verblockung der Filteroberfläche kaum entgegengewirkt werden kann. Gleichzeitig gibt es derzeit keine hermetisch abgeschlossenen Einweg-Tiefenfiltrationssysteme, wie man sie seitens der Anwender gerne einsetzen würde, um den Produktkontakt mit der Umgebung oder gar dem Bedienerpersonal auszuschließen. D.h. die Tiefenfilter-Technologie, die ohnehin technischen Einschränkungen unterlegen ist, wird mit dem oben beschriebenen Trend der Intensivierung neben mangelnden praktischen Eigenschaften auch zunehmend an ihre wirtschaftlichen Grenzen geführt.

In anderen Industriezweigen, wie beispielsweise in der Getränkeindustrie, hat sich bei ähnlicher Problemstellung die Querstromfiltration als eine geeignete Möglichkeit erwiesen, die Zellen gegenüber den konventionellen Methoden effizient und zugleich wirtschaftlich abzutrennen. Dabei kann die Querstromfiltration häufig mit einem einzigen Prozessschritt gleich zwei oder mehrere traditionelle Klärschritte ersetzen, nämlich die Zentrifugation und oft auch eine darauffolgende mehrstufige Tiefenfiltration. Als besonders geeignet für die Zellabtrennung mittels Querstromfiltration erweisen sich Hohlfasermodule mit Ultra- oder Mikrofiltrationsmembranen.

Obwohl die Querstromfiltration mit Mikrofiltrationsmembranen als etabliertes Trennverfahren weit verbreitet und hinlänglich bekannt ist, stellen in dem hier interessierenden Anwendungsgebiet der in der Fermentationsbrühe enthaltene Zellanteil in Form der sogenannten Biofeuchtmasse sowie die zu erreichende Proteinausbeute eine besondere Herausforderung dar. Denn mit zunehmender Abtrennung von geklärtem Protein-Filtrat (Permeat) aus der ursprünglichen Fermenterlösung steigt die Konzentration der im Bioreaktor vorliegenden Biofeuchtmasse sukzessive an und führt im Verlauf der Filtration zur Erhöhung der Viskosität. Dieser Prozess lässt sich aufgrund der technischen Rahmenbedingungen nicht unbegrenzt fortführen. Daher ist es wichtig, die technischen Rahmenbedingungen so zu gestalten bzw. zu optimieren, dass die Ausbeute maximiert werden kann.

Die Rahmenbedingungen stellen ein Zusammenspiel von verschieden Faktoren dar, welche die Wirtschaftlichkeit eines Trennverfahrens stark beeinflussen können. Im Wesentlichen sind dies die geometrische Ausgestaltung der Filtrationseinheit (hier insbesondere ein Hohlfasermodul) die Systembedingungen, wie Pumpensystem, sowie der sich dynamisch ändernde Flüssigkeitszustand (z.B. Viskosität und Temperatur). Bei zunehmender Konzentrierung und steigender Viskosität ist der freie Durchfluss durch die röhrenförmigen Kanäle des Hohlfasermoduls nicht mehr oder nur noch begrenzt möglich, so dass die Filtrationsleistung stark zurückgehen kann und eine optimale Überströmung der Membranoberfläche zunehmend schwierig wird. Dies kann so weit gehen, dass die Filtration in Bezug auf spezifische Flussleistung und eingebrachte Pumpenenergie nicht mehr wirtschaftlich zu gestalten ist bzw. sich ein totaler Einbruch der Filtrationsleistung einstellt und der Prozess abgebrochen werden muss.

Um einen solchen Eskalationspunkt zu vermeiden, könnte zum Beispiel eine kontrollierte Verdünnung mit Puffer oder Substrat (Nährlösung) helfen, die Flüssigkeit auf einem systemtechnisch geeigneten Viskositätsniveau zu halten. Dies hat jedoch wiederum den Nachteil, dass dadurch die Lösung, in der das Zielprotein enthalten ist, ein immer größeres Volumen einnimmt, was ebenfalls in den weiteren Aufreinigungsschritten, die dem ersten Klärschritt nachgeschaltet sind, von Nachteil ist.

In der hier vorwiegend interessierenden Anwendung ist also der Erfolg bzw. Misserfolg der jeweiligen Zellabtrenntechnologie neben der technischen Machbarkeit vor allem auch durch die zu erzielenden Proteinausbeute - als wertgebenden Bestandteil - gekennzeichnet. Das heißt also, es muss eine optimale Technologie, verbunden mit einem geeigneten Verfahren, gefunden werden, die es ermöglicht, die maximale Proteinausbeute ohne negative Beeinflussung der noch folgenden Schritte im Reinigungsprozess des biopharmazeutischen Zielporteins zu erzielen.

Der oben beschriebene optimale Betriebspunkt kann dadurch erreicht werden, dass zunächst die Zellabtrennung mit der Querstromfiltration in klassischer Art und Weise, also mit Abtrennung der Proteinlösung bei gleichzeitiger Aufkonzentrierung der Biomasse, durchgeführt wird. Sobald durch die Abtrennung der klaren zellfreien Lösung ein kritischer Viskositätswert oder eine Filtratflussuntergrenze erreicht wird, erfolgt eine Verdünnung durch Zufuhr einer Verdünnungsflüssigkeit in das System. In bekannten Querstromfiltrations-Verfahren erfolgt diese Zufuhr der Verdünnungsflüssigkeit in der Regel direkt in den Vorlage- oder Rezirkulationstank, oder aber in die Querstromfiltrations-Kreislaufleitung.

Zur Erhaltung bzw. Optimierung des Filtrationsflusses durch die Membran wurden bereits Querstromfiltrationsprozesse vorgestellt, bei denen eine Rückspülung mit Filtrat erfolgt, welches soeben die Membran durchdrungen hat. Entsprechende Systeme sind z.B. in der US 6 544 424 B1 und der WO 2018/022661 A1 beschrieben. Bei der auch als "Alternating Tangential Flow (ATF) Filtration" bezeichneten Technologie wird der sich aufbauende Oberflächenbelag (Fouling) durch die Umkehrung der Flussrichtung und damit auch der Druckverhältnisse zwischen mittlerem Eingangsdruck auf der Membran-Anströmseite und Ausgangdruck auf der Membran-Abströmseite, gelockert oder gelöst und im Idealfall mit dem Retentatstrom mitgerissen und abtransportiert. Im Idealfall lässt sich dadurch der Filtratfluss (Flux) vollständig oder zumindest teilweise wieder auf einen anfänglichen Flusswert steigern oder zumindest für längere Zeit auf einem hohen Niveau halten. Der Nachteil dieser Verfahrensweise ist jedoch, dass die Rückspülung mit Filtrat die effektive Filtrationsleistung und damit die Effizienz des Gesamtsystems negativ beeinflusst, da das gerade zurückgeförderte Filtrat nach der Rückspülung ja erneut von der Anströmseite der Membran zur Filtratseite geführt werden muss.

Vor diesem Hintergrund ist es die Aufgabe der Erfindung, eine Technologie zur Zellabtrennung bereitzustellen, die es ermöglicht, eine möglichst hohe Ausbeute eines biopharmazeutischen Zielporteins ohne negative Beeinflussung der noch folgenden Schritte im Reinigungsprozess des Proteins zu erzielen. Insbesondere soll mit einer solchen Technologie unter Verwendung von Mikrofiltrationsmembranen, insbesondere im Hohlfaserformat, aber auch in anderen Formaten wie Platten-Rahmen-Module, Röhrenmodule, Spiralwickelmodule etc., ein sowohl technisch als auch wirtschaftlich optimaler Betriebspunkt einstellbar sein, vor allem hinsichtlich Produktausbeute, Produktqualität und Verdünnungsgrad der Lösung.

Gelöst wird diese Aufgabe durch eine Vorrichtungsanordnung mit den Merkmalen des Anspruchs 1 sowie durch ein Verfahren mit den Merkmalen des Anspruchs 13. Vorteilhafte und zweckmäßige Ausgestaltungen der erfindungsgemäßen Vorrichtungsanordnung und des erfindungsgemäßen Verfahrens sind in den zugehörigen Unteransprüchen angegeben.

Die erfindungsgemäße Vorrichtungsanordnung zur Abtrennung von Zellen aus einem Kulturmedium in einem Bioprozess, insbesondere in einem biopharmazeutischen Prozess, umfasst Folgendes:
- einen Vorratsbehälter für das Kulturmedium, insbesondere einen Bioreaktor;
- ein Filtrationsmodul, insbesondere ein Hohlfasermodul, mit wenigstens einer überströmbaren Membran;
- eine Zulaufleitung, die den Vorratsbehälter mit einem Unfiltrat-Einlass des Filtrationsmoduls verbindet, zum Zuführen von Kulturmedium in das Filtrationsmodul;
- eine Rücklaufleitung, die einen retentatseitigen Auslass des Filtrationsmoduls mit dem Vorratsbehälter verbindet, zum Rückführen von Retentat in den Vorratsbehälter;
- eine Filtratleitung, die an einen permeatseitigen Auslass des Filtrationsmoduls angeschlossen ist, zum Abführen von Filtrat;
- eine erste Pumpe zur Aufrechterhaltung eines Rezirkulationskreislaufs;
- eine Rückspülleitung, die zu einem (anderen) permeatseitigen Anschluss und/oder zum permeatseitigen Auslass des Filtrationsmoduls führt, zum Zuführen einer Spülflüssigkeit in das Filtrationsmodul; und
- eine zweite Pumpe zum Fördern der Spülflüssigkeit in das Filtrationsmodul.

Mit der erfindungsgemäßen Vorrichtungsanordnung kann der Zellabtrennungsschritt unabhängig von der Art des jeweiligen Fermentationsprozesses optimal gestaltet werden, um sowohl Proteinausbeute als auch die Prozesskosten unter dynamischen, also sich verändernden Rahmenbedingungen zu optimieren. Insbesondere ist es mit dem erfindungsgemäßen Aufbau möglich, eine während der Filtration erforderliche oder gewünschte Verdünnung des Kulturmediums vorteilhaft mit einer Reinigung der Membranoberfläche zu verknüpfen. Dank der separaten Rückspülleitung und der zweiten Pumpe kann während der Filtration eine Spülflüssigkeit, mit der einerseits die sich im Laufe der Filtration bildende Deckschicht oder andere Rückstände (Fouling) von der Membran gelockert bzw. gelöst werden können, zusätzlich in den Rezirkulationskreislauf eingebracht werden, um so andererseits zur Verdünnung des zirkulierenden Kulturmediums beizutragen. Die Spülflüssigkeit kann also mehrere Funktionen gleichzeitig erfüllen, nämlich (i) eine Reinigung der Membran, um einer Verblockung des Filters entgegenzuwirken und die Filtrationsleistung so gut wie möglich aufrechtzuerhalten, und (ii) eine Verdünnung des Kulturmediums, um dieses auf einem systemtechnisch geeigneten Viskositätsniveau zu halten, und/oder (iii) eine optimale Steuerung der Zellernährung während der Fermentation, worauf später noch genauer eingegangen wird.

Als Spülflüssigkeit eignet sich insbesondere eine auf das Kulturmedium abgestimmte Pufferlösung. Bei konventionellen Systemen ist das Einbringen einer solchen Pufferlösung allenfalls zum Zwecke der Verdünnung des Kulturmediums während der Filtration vorgesehen. Gemäß der vorliegenden Erfindung wird die Pufferlösung darüber hinaus gleichzeitig zur Reinigung der Membran des Filtrationsmoduls genutzt. Eine Membraneinigung bei laufender Filtration ist bei konventionellen Systemen entweder gar nicht vorgesehen oder erfolgt, wie bei den eingangs genannten ATF-Systemen, durch eine Strömungsumkehr, wobei die gelösten Deckschichtbestandteile mit dem Unflitratfluss aus dem Filtrationsmodul in den Vorratsbehälter (Bioreaktor) gespült werden.

Alternativ kann statt einer Pufferlösung auch reines Wasser (WFI), das kostengünstiger als eine Pufferlösung ist, oder eine andere geeignete Flüssigkeit als Spülflüssigkeit verwendet werden.

Gemäß einem besonderen Aspekt der Erfindung erfolgt die Rückspülung mit Substrat, also einer für die Kultivierung der Zellen im Bioreaktor vorgesehenen Nährlösung. Die Rückspülung mit Substrat eignet sich insbesondere für einen Fed-Batch- oder einen kontinuierlichen Prozess (Perfusionsprozess). In einem solchen Prozess muss ohnehin Substrat in den Bioreaktor nachgefördert werden, um die Bedingungen für das Zellwachstum zu verbessern und den Volumenverlust durch das fortgesetzte Abführen von filtrierter Proteinlösung auszugleichen. Das Substrat kann also gleichzeitig als Spülflüssigkeit zur Reinigung der Membran und als Nährlösung für die Zellkultivierung genutzt werden.

Bei der erfindungsgemäßen Vorrichtungsanordnung ist vorzugsweise ein erstes Ventil in der Rücklaufleitung zum selektiven Sperren des Retentatrücklaufs in den Vorratsbehälter vorgesehen, welches während der Filtration als Drossel- oder Druckregelventil eingesetzt wird, um den Transmembrandruck (TMP) als treibende Kraft zu steuern und zu regeln. Daneben kann ein zweites Ventil, das in der Filtratleitung zum selektiven Sperren der Abfuhr des Filtrats vorgesehen ist, dazu genutzt werden, den Abfluss aus dem Filtrationsmodul während eines Rückspülvorgangs zu unterbinden.

Durch ein drittes Ventil in der Rückspülleitung kann die Spülflüssigkeitszufuhr zum Filtrationsmodul gesteuert werden.

Im Gegensatz zur Anordnung der Membranpumpe bei den bekannten ATF-Systemen ist hier die erste Pumpe, die zur Aufrechterhaltung des Rezirkulationskreislaufs dient, vorzugsweise in die Zulaufleitung integriert, d.h. sie ist stromauf des Filtrationsmoduls angeordnet.

Für den Fall, dass die erfindungsgemäße Vorrichtungsanordnung nicht für einen Batch-Prozess, sondern für einen Fed-Batch- oder einen kontinuierlichen Prozess (Perfusionsprozess) ausgelegt werden soll, ist das Vorsehen einer Substratzuführleitung zum Zuführen von Substrat in den Vorratsbehälter erforderlich, sofern dieser gleichzeitig als Bioreaktor dient, in dem die Zellen kultiviert werden.

Gemäß einer ersten Variante kann diese Substratzuführleitung als separate Leitung direkt in den Vorratsbehälter münden. In diesem Fall ist dann eine dritte Pumpe zum Fördern des Kulturmediums in den Vorratsbehälter vorgesehen.

Gemäß einer alternativen zweiten Variante münden die Substratzuführleitung und die Rückspülleitung stromauf der zweiten Pumpe in eine gemeinsame Zuführleitung, die wiederum in den ansonsten ungenutzten permeatseitigen Anschluss und/oder in den permeatseitigen Auslass des Filtrationsmoduls mündet. Diese Aufbauvariante ermöglicht es, die zweite Pumpe, die insbesondere in die gemeinsame Zuführleitung integriert sein kann, sowohl für die Zufuhr von Substrat als auch für die Zufuhr von Pufferlösung zu nutzen. Deshalb kann bei dieser Variante auf eine dedizierte Pumpe zum Fördern des Substrats verzichtet werden. Dadurch vereinfacht sich das Design der Vorrichtungsanordnung, und sie kann kostengünstiger gestaltet werden.

Mithilfe des dritten Ventils in der Rückspülleitung und einem zusätzlichen vierten Ventil in der Substratzuführleitung kann je nach Bedarf umgeschaltet werden zwischen Spülflüssigkeitszufuhr und Substratzufuhr, oder beide Leitungen können gesperrt werden.

Gemäß einer besonderen Ausführungsform der erfindungsgemäßen Vorrichtungsanordnung ist in der Rückspülleitung bzw. in der gemeinsamen Zuführleitung ein Sterilfilter angeordnet. Dadurch kann verhindert werden, dass im Zuge der Spülflüssigkeits- bzw. Substratzufuhr Verunreinigungen von außen in den Rezirkulationskreislauf eingetragen werden. Dies ist besonders bei Perfusionsprozessen von Bedeutung, die über einen vergleichsweise langen Zeitraum laufen, z.B. mehrere Wochen. Eine Kontamination hätte hier besonders schwerwiegende Folgen. Aber auch bei Batch- oder Fed-Batch-Prozessen ist das Vorsehen eines Sterilfilters in der Substratzuführleitung sinnvoll, da eine Kontamination immer zu vermeiden ist.

Um das Potential der erfindungsgemäßen Vorrichtungsanordnung voll auszuschöpfen, ist es vorteilhaft, Sensoren und eine Steuereinheit zur direkten oder indirekten Bestimmung und Überwachung eines oder mehrerer der folgenden Parameter vorzusehen: Viskosität; elektrische Leitfähigkeit; Fluss (insbesondere Rezirkulationsfluss und/oder Filtratfluss); Druck, insbesondere Transmembrandruck (TMP). Die Steuereinheit ist in diesem Fall dazu eingerichtet, eine Rückspülung in Abhängigkeit des Erreichens eines oder mehrerer vorbestimmter Grenzwerte der genannten Parameter auszulösen und zu steuern. Auf diese Weise lässt sich der gesamte Prozess fortwährend optimal einstellen und steuern. Besonders vorteilhaft ist eine halb- oder sogar vollautomatische Steuerung des Prozesses, wenn die Steuereinheit z.B. die einzelnen Pumpen und Ventile der Vorrichtungsanordnung ansteuern kann. Insbesondere können anhand der Parameter optimale Zeitpunkte für die Spülvorgänge mit gleichzeitiger Verdünnung bestimmt werden, und die Dauer, der Druck und die Spülflüssigkeitsmenge der Spülvorgänge kann ebenfalls optimal eingestellt werden, um gewünschte Leistungswerte des Prozesses (Filtrationsleistung, Verdünnungsgrad etc.) zu erreichen bzw. zu erhalten.

Die Erfindung schafft auch ein Verfahren zur Abtrennung von Zellen aus einem Kulturmedium, insbesondere unter Verwendung einer erfindungsgemäßen Vorrichtungsanordnung. Das erfindungsgemäße Verfahren weist folgende Schritte auf:
- Kultivieren von Zellen in einem Kulturmedium in einem Bioreaktor;
- Filtrieren des Kulturmediums in einem Filtrationsmodul mit wenigstens einer Membran, insbesondere in einem Hohlfasermodul, wobei das Kulturmedium einen Rezirkulationskreislauf durchläuft;
- Spülen der Membran mit einer Spülflüssigkeit während des Filtrierens, wobei die Spülflüssigkeit insbesondere eine Pufferlösung oder Wasser oder Substrat ist, und wobei durch das Spülen Spülflüssigkeit in den Rezirkulationskreislauf eingebracht wird.

Entsprechend den obigen Erläuterungen bedeutet "Spülen während des Filtrierens", dass die Spülvorgänge in den übergeordneten Schritt des Filtrierens eingebettet sind. Je nach Bedarf kann dabei der Rezirkulationskreislauf für einen Spülvorgang vollständig unterbrochen oder eingeschränkt aufrechterhalten werden. Während die Permeatabfuhr während eines Spülvorgangs in jedem Fall geschlossen sein sollte, kann die Kreislaufpumpe (erste Pumpe) weiterlaufen, ggf. mit gedrosselter Leistung, oder auch vollständig abgeschaltet sein. Der Retentatrücklauf in den Vorratsbehälter sollte während des Spülvorgangs offen sein, zumindest dann, wenn die Kreislaufpumpe bei der Rückspülung nicht gestoppt wurde. Während der eigentlichen Filtration dient das im Retentatrücklauf angeordnete Ventil zur Regelung des Transmembrandrucks und kann dabei teilweise bzw. zeitweise geschlossen sein.

Um eine unabhängige Zufuhr von Spülflüssigkeit in das Filtrationsmodul zu ermöglichen, ist vorzugsweise vorgesehen, dass diese über eine separate Leitung in einen oder mehrere permeatseitige(n) Anschluss bzw. Anschlüsse des Filtrationsmoduls gefördert wird, der/die von dem Unfiltrat-Einlass des Filtrationsmoduls getrennt ist/sind, in den das zu filtrierende Kulturmedium aus dem Vorratsbehälter gefördert wird.

Für eine optimale Einstellung und Steuerung des Prozesses, einschließlich der gleichzeitig durchgeführten Spül- und Verdünnungsvorgänge, empfiehlt es sich, zumindest während des Filtrierens einen oder mehrere der folgenden Parameter zu bestimmen und zu überwachen: Viskosität; elektrische Leitfähigkeit; Fluss (insbesondere Rezirkulationsfluss und/oder Filtratfluss); Druck, insbesondere Transmembrandruck. Eine Rückspülung wird dann, wie oben bereits beschrieben, in Abhängigkeit des Erreichens eines oder mehrerer vorbestimmter Grenzwerte der genannten Parameter ausgelöst und gesteuert.

Der Schritt des Spülens kann mehrfach nacheinander durchgeführt werden und/oder während des übergeordneten Schritts des Filtrierens zyklisch wiederholt werden, wobei die zeitlichen Abstände zwischen den Spülvorgängen variieren können, insbesondere in Abhängigkeit der von den Sensoren fortwährend ermittelten Parameter.

Bei den Spülvorgängen kann auch die Spülflüssigkeit variiert werden. Entweder nach einem vorgegebenen Schema oder nach einem anhand der zuvor genannten Parameter ermittelten Bedarf kann zwischen Pufferlösung, reinem Wasser und Substrat oder zumindest zwischen zwei dieser flüssigen Medien gewechselt werden. Grundsätzlich können auch noch weitere Spülflüssigkeiten einbezogen werden.

Bei Anwendung des erfindungsgemäßen Verfahrens in einem Batch-Prozess werden die Schritte des Kultivierens und des Filtrierens nacheinander durchgeführt.

Im Falle eines Fed-Batch- oder Perfusionsprozesses werden die Schritte des Kultivierens und des Filtrierens dagegen zumindest teilweise gleichzeitig durchgeführt.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 einen schematischen Aufbau einer Vorrichtungsanordnung gemäß einer ersten Ausführungsform der Erfindung;
- Figur 2 einen schematischen Aufbau einer Vorrichtungsanordnung gemäß einer zweiten Ausführungsform der Erfindung; und
- Figur 3 einen schematischen Aufbau einer Vorrichtungsanordnung gemäß einer dritten Ausführungsform der Erfindung.

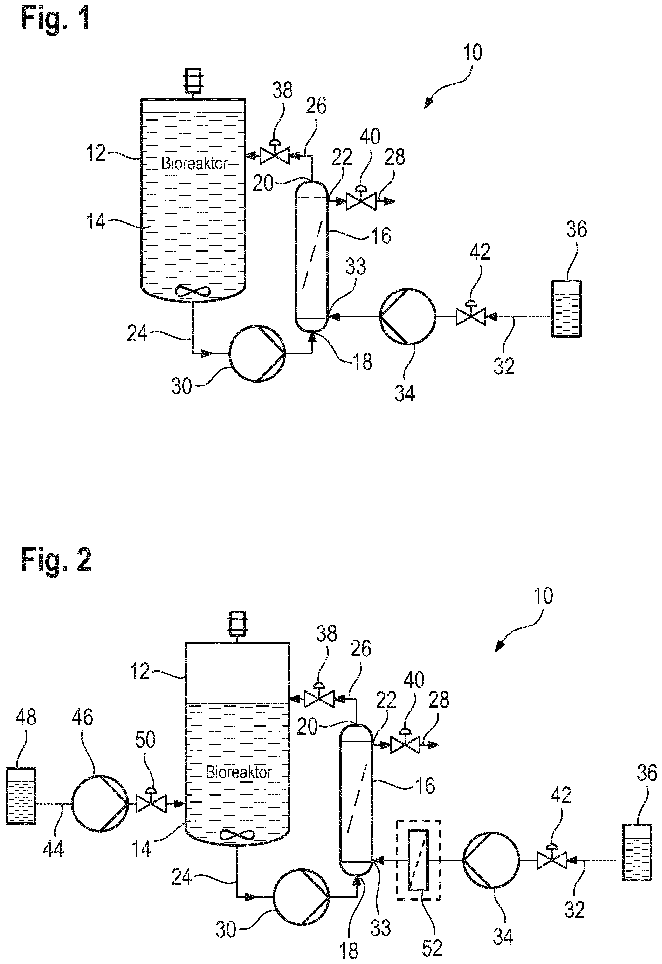

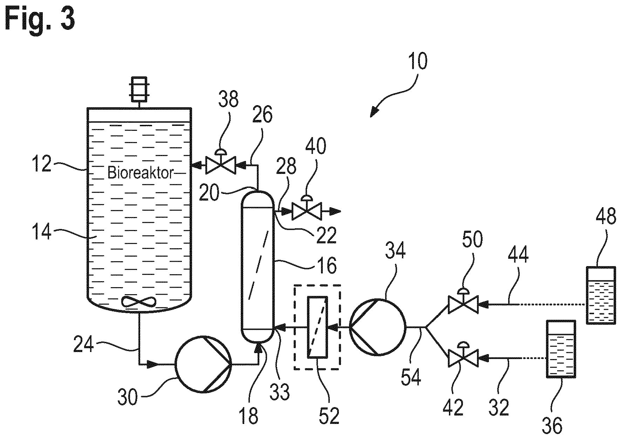

Im Folgenden werden anhand der Figuren 1 bis 3 exemplarisch drei verschiedene Aufbauten einer Vorrichtungsanordnung 10 beschrieben, mit der im Rahmen eines biopharmazeutischen Fermentationsprozesses Zellen aus einem Kulturmedium abgetrennt werden. Für einander entsprechende Komponenten werden die gleichen Bezugszeichen verwendet, wobei bezüglich der in den Figuren 2 und 3 gezeigten Aufbauten nur auf die wesentlichen Unterschiede zu dem in Figur 1 gezeigten Aufbau eingegangen wird.

Gemäß dem in Figur 1 dargestellten Aufbau umfasst die Vorrichtungsanordnung 10 einen Vorratsbehälter 12 für ein Kulturmedium 14 und ein Filtrationsmodul 16, mit dem zur Gewinnung eines Zielproteins Zellen aus dem Kulturmedium 14 abgetrennt werden können. Bei dem Vorratsbehälter 12 handelt es sich um einen Bioreaktor, in dem der Fermentationsprozess zur Kultivierung der Zellen stattfindet.

Das Filtrationsmodul 16 ist hier ein Hohlfasermodul mit wenigstens einer überströmbaren Membran, d.h. das Filtrationsmodul 16 ist für eine Querstromfiltration ausgelegt. Dementsprechend weist das Filtrationsmodul 16 wenigstens drei Anschlüsse auf, nämlich einen Unfiltrat-Einlass 18 für das Unfiltrat aus dem Vorratsbehälter 12, einen retentatseitigen Auslass 20 und einen permeatseitigen Auslass 22. Bei vielen Hohlfasermodulen ist noch ein weiterer permeatseitiger Anschluss 33 vorgesehen, der bei manchen klassischen Filtrationsanwendungen nicht genutzt und deshalb mit einem Stopfen verschlossen wird. Auf die besondere Nutzung dieses Anschlusses 33 im Zusammenhang mit der Erfindung wird später noch genauer eingegangen.

Eine Zulaufleitung 24 verbindet den Vorratsbehälter 12 mit dem Einlass 18 des Filtrationsmoduls 16. Eine Rücklaufleitung 26 verbindet den retentatseitigen Auslass 20 des Filtrationsmoduls 16 mit dem Vorratsbehälter 12. An den permeatseitigen Auslass 22 des Filtrationsmoduls 16 ist eine Filtratleitung 28 angeschlossen.

In die Zulaufleitung 24 ist eine erste Pumpe 30 integriert, mittels der ein Rezirkulationskreislauf des Unfiltrats aufrechterhalten werden kann. Bei diesem Rezirkulationskreislauf wird Kulturmedium 14 vom Vorratsbehälter 12 durch die Zulaufleitung 24 zum Unfiltrat-Einlass 18 des Filtrationsmoduls 16 gefördert. Im Filtrationsmodul 16 überströmt das Kulturmedium 14 die Membran, die durchlässig für das Zielprotein ist, nicht jedoch für die Zellen. Während die von Zellen befreite Lösung (Permeat bzw. Filtrat) über den permeatseitigen Auslass 22 abgeführt wird, gelangt das Retentat über den Auslass 24 und die Rücklaufleitung 26 zurück in den Vorratsbehälter 12. Die erste Pumpe 30 könnte dafür grundsätzlich auch in die Rücklaufleitung 26 integriert sein, wobei dann kein positiver Transmembrandruck aufgebaut werden könnte.

Des Weiteren führt eine Rückspülleitung 32 zu einem permeatseitigen Anschluss 33 des Filtrationsmoduls 16. In die Rückspülleitung 32 ist eine zweite Pumpe 34 integriert, mittels der eine Spülflüssigkeit aus einem Reservoir 36 zum permeatseitigen Anschluss 33 des Filtrationsmoduls 16 gefördert werden kann. Das Reservoir 36 ist insbesondere mit einer Pufferlösung oder Wasser für Injektionszwecke (WFI) gefüllt; es kann aber auch eine andere geeignete Spülflüssigkeit verwendet werden.

In die Rücklaufleitung 26 ist ein erstes Ventil 38 integriert, mit dem der Retentatrücklauf in den Vorratsbehälter 12 gesperrt oder gedrosselt werden kann. Ein zweites Ventil 40 ist in die Filtratleitung 28 integriert. Mit diesem zweiten Ventil 40 kann die Abfuhr des Filtrats (zellbefreites Kulturmedium) gesperrt oder gedrosselt werden. Ein in die Rückspülleitung 32 integriertes drittes Ventil 42 dient zum selektiven Sperren oder Drosseln der Spülflüssigkeitszufuhr zum permeatseitigen Anschluss 33 des Filtrationsmoduls 16.

Vorzugsweise sind alle Komponenten der Vorrichtungsanordnung 10, insbesondere das Filtrationsmodul 16, die Leitungen 24, 26, 28, 32 und die Ventile 38, 40, 42 sowie zumindest die Medium- bzw. Spülflüssigkeit-berührenden Teile der Pumpen 30, 34 als Einweg-Komponenten ausgelegt. Das bedeutet, dass diese Komponenten vornehmlich aus sterilisierbaren bzw. autoklavierbaren Kunststoffen bestehen. Die Komponenten können vor Gebrauch vormontiert, sterilisiert und mittels geeigneter Sterilkonnektoren steril an den Bioreaktor angeschlossen werden. Nach einmaligem Gebrauch werden alle Einweg-Komponenten vollständig entsorgt.

Der in Figur 1 gezeigte Aufbau eignet sich insbesondere für einen sogenannten Batch-Prozess, bei dem nach dem initialen Füllen des Bioreaktors, der gleichzeitig als Vorratsbehälter 12 dient, und dem Beginn der Kultivierung keine weitere Zugabe von Substrat mehr vorgesehen ist.

Der Bioreaktor wird bis auf das Zielvolumen mit Substrat befüllt und mit Zellen angeimpft (Inokulation). Sobald die optimalen Zell- bzw. Proteinkonzentrationen (Titer) erreicht sind, erfolgt der Prozessschritt der Zellabtrennung bzw. der Ernte des Zielproteins. Dabei soll die Proteinausbeute so hoch wie möglich gestaltet werden, und die Zellen (Biomasse) sollen vollständig von der Protein beinhaltenden Lösung getrennt werden.

Bei den konventionellen Technologien wie der Zentrifugation und/oder der Tiefenfiltration erreicht man hierbei in der Regel Protein-Ausbeuteraten von lediglich 85 % bzw. teilweise sogar noch darunter. Gleichzeitig ist aufgrund verfahrenstechnischer Gegebenheiten auch eine Verdünnung nicht zu vermeiden. Diese liegt je nach Prozessbedingungen und Ausbeuteabsichten bei bis zu 30 %. Beide Parameter, Ausbeute und Verdünnung, sind bei den konventionellen Verfahren nur bedingt beinflussbar. Mit der in biotechnologischen Fermentationsprozesse deutlichen steigenden Tendenz zur Intensivierung und Titer- und damit Ausbeuteerhöhung steigt der Materialeinsatz bei diesen konventionellen Trennverfahren exorbitant an und wird mittlerweile wirtschaftlich immer unattraktiver.

Mit dem oben beschriebenen Aufbau der Vorrichtungsanordnung 10 und der nachfolgend beschriebenen Vorgehensweise während des Filtrierens eröffnen sich diesbezüglich neue Möglichkeiten der Optimierung, wobei der erforderliche Platzbedarf sowie der technologische Aufwand nicht so stark von Titer und Zellkonzentration abhängig sind.

Gemäß dem hier vorgeschlagenen Verfahren erfolgt die Filtration in dem oben bereits beschriebenen Rezirkulationskreislauf, in dem das Kulturmedium 14 das Filtrationsmodul 16 wiederholt durchläuft. Ab einem gewünschten und evtl. im Vorfeld ermittelten Betriebspunkt (insbesondere bezüglich Viskosität, Fluss (Rezirkulationsfluss und/oder Filtratfluss), Transmembrandruck Verdünnung) wird die Filtration bzw. Konzentrierung durch zyklisches Rückspülen unterbrochen. Dazu wird mit Hilfe der zweiten Pumpe 30 Spülflüssigkeit aus dem Reservoir 36 zum permeatseitigen Anschluss 33 des Filtrationsmoduls 16 gefördert. Die durch die Membran auf die Retentatseite strömende Spülflüssigkeit sorgt für eine Reinigung der Membran, wie weiter unten noch genauer erläutert wird.

Als Spülflüssigkeit eignet sich insbesondere eine Pufferlösung, die ohnehin im Rahmen des Fermentationsprozesses zum Einsatz kommt. Eine solche Pufferlösung verhält sich also neutral und erhöht nur das Volumen im Rezirkulationskreislauf, was aber angesichts der durch die Abfuhr des Permeats ansteigenden Viskosität des Kulturmediums gerade erwünscht ist. Somit lassen sich die Prozess(teil)schritte "Verdünnung des Kulturmediums" und "Reinigung der Membran im Filtrationsmodul 16" während des Filtrierens zu einem einzigen Schritt kombinieren ("2 in 1").

Alternativ kann auch mit reinem Wasser (WFI) gespült werden, oder es kann zwischen Pufferlösung und Wasser gewechselt werden.

Je nachdem, welche Differenzdruckbedingungen erzielt werden sollen, kann während der Rückspülung die erste Pumpe 30 weiterlaufen, gedrosselt oder auch abgeschaltet werden. Während des Rückspülvorgangs ist das zweite Ventil 40 in der Filtratleitung 28 grundsätzlich geschlossen und das erste Ventil 38 in der Rücklaufleitung 26 offen. Dabei entsteht ein Differenzdruck an der Membranoberfläche, der dem Filtrationsstrom entgegenwirkt, wodurch die Deckschicht auf der Membran vollständig oder teilweise abgelöst wird. Dadurch wird einem Verblocken des Filters (Fouling) effektiv entgegengewirkt, was zu einer zumindest wieder zeitweisen Erhöhung der zuvor gesunkenen Filtrationsleistung führt.

Im Anschluss an die Rückspülung wird die Filtration weiter fortgesetzt. Der Rückspülvorgang kann beliebig oft wiederholt bzw. beliebig lange durchgeführt werden, bis die jeweiligen Grenzwerte in Bezug auf Ausbeute, Verdünnung und/oder Prozesszeit erreicht sind.

Durch die Verwendung von geeigneten Sensoren aus bekannten Querstromfiltrationssystemen, die als Einwegsensoren in die Vorrichtungsanordnung 10 eingebunden werden können, ist es möglich, die Filtrationsleistung permanent zu messen und mit Hilfe einer Steuereinheit die idealen Parameter (insbesondere Zeitpunkt, Dauer, Volumen, Druck, Geschwindigkeit) der Rückspülung zu ermitteln und entsprechend einzustellen bzw. im Prozess zu verändern und zu optimieren, sodass die Filtrationsleistung über die Prozessdauer insgesamt bestmöglich aufrechterhalten wird.

Bei dem in Figur 2 gezeigten Aufbau der Vorrichtungsanordnung 10 ist zusätzlich eine Substratzuführleitung 44 vorgesehen, die in den Vorratsbehälter 12 mündet. In die Substratzuführleitung 44 ist eine dritte Pumpe 46 integriert, sodass Substrat, z.B. aus einem Tank 48, in den Bioreaktor gefördert werden kann. Mittels eines in die Substratzuführleitung 44 integrierten vierten Ventils 50 kann die Zufuhr von Substrat selektiv gesperrt oder gedrosselt werden. Optional kann ein Sterilfilter 52 in die Rückspülleitung 32 integriert sein. Das Vorsehen eines solchen Sterilfilters ist auch in der Substratzuführleitung 44 sinnvoll, um von dieser Seite die Sterilität im Bioreaktor sicher aufrechtzuerhalten.

Der in Figur 2 gezeigte Aufbau eignet sich insbesondere für einen sogenannten Fed-Batch- oder einen kontinuierlichen Prozess (Perfusionsprozess), bei denen entweder in einem Schritt, zyklisch oder auch kontinuierlich im Verlaufe des Fermentationsprozesses frisches Substrat in den Bioreaktor zugegeben wird, um die Lebensbedingungen für die Zellen und damit die Proteinausbeute zu steuern und positiv zu beeinflussen. Während beim Fed-Batch Prozess die Zellen am Ende der Kultivierung in einem einmaligen Schritt vom Zielprotein getrennt werden, erfolgt beim Perfusionsprozess dieser Schritt bereits im Verlauf der Kultivierung selbst, sozusagen in einem semi-kontinuierlichen bzw. kontinuierlichen Modus. Am Ende des Fed-Batch Verfahrens bzw. im Verlauf der Perfusion werden wiederum die konventionellen Technologien Zentrifugation und/oder Tiefenfiltration oder aber auch die hier interessierende Querstromfiltration zur Zellabtrennung eingesetzt. Dabei werden im Verlaufe der Kultivierung Teilmengen geerntet, um dann am Ende eine finale Aufkonzentrierung zur Ausbeutesteigerung anzuschließen.

Mit dem in Figur 2 gezeigten Aufbau der Vorrichtungsanordnung 10 und der nachfolgend beschriebenen Vorgehensweise bei der Zellabtrennung eröffnen sich auch in Bezug auf Fed-Batch- und Perfusionsprozesse neue Möglichkeiten der Optimierung, wobei der erforderliche Platzbedarf sowie der technologische Aufwand nicht so stark von Titer und Zellkonzentration abhängig sind.

Die Zugabe von Kulturmedium 14 beim Fed-Batch- bzw. Perfusions-Prozess und dessen im Rahmen der Optimierung des laufenden Prozesses vorgesehene Verdünnung mit Pufferlösung und/oder reinem Wasser können ebenfalls mit der Rückspülung zur Membranreinigung kombiniert werden. Wie oben beschrieben wird ab dem gewünschten Betriebspunkt (insbesondere bezüglich. Viskosität, Fluxleistung, Verdünnung) das Filtrieren durch zyklisches Rückspülen und damit Reinigen der Membranoberfläche mit einer geeigneten Pufferlösung und/oder reinem Wasser unterbrochen. Dabei erfolgt jedoch die Zufuhr von Substrat über die separate Substratzuführleitung 44 und die dritte Pumpe 46 direkt in den Bioreaktor, während die Zufuhr der Pufferlösung und/oder reinem Wasser - und die gleichzeitig stattfindende Membranrückspülung - über die Rückspülleitung 32 und die zweite Pumpe 34 in den permeatseitigen Anschluss 33 des Filtrationsmoduls 16 erfolgt.

Figur 3 zeigt einen alternativen Aufbau der Vorrichtungsanordnung 10, der sich ebenfalls für einen Fed-Batch- oder Perfusionsprozess eignet. Der Aufbau entspricht großteils dem in Figur 2 gezeigten Aufbau, unterscheidet sich aber in der Art der Substratzufuhr, wie nachfolgend kurz beschrieben wird.

Die Substratzuführleitung 44 mündet hier nicht direkt in den Bioreaktor, sondern die Substratzuführleitung 44 und die Rückspülleitung 32 münden beide in eine gemeinsame Zuführleitung 54, die wiederum in den permeatseitigen Anschluss 33 des Filtrationsmoduls 16 mündet. Die zweite Pumpe 34 und der optionale Sterilfilter 52 sind in diese gemeinsame Zuführleitung 54 integriert.

Mit diesem Aufbau ist es möglich, die Zufuhr von Substrat und die Zufuhr der Spülflüssigkeit mit derselben Pumpe zu bewerkstelligen, nämlich mit der zweiten Pumpe 34. Die Zufuhr von Substrat erfolgt bei dieser Aufbauvariante nicht direkt in den Bioreaktor, sondern indirekt über den permeatseitigen Anschluss 33 des Filtrationsmoduls 16, durch die Membran hindurch und über die Rücklaufleitung 26. Durch entsprechende Schaltung des dritten Ventils 42 und des vierten Ventils 50 kann - je nach Prozess(teil)schritt - wahlweise Substrat aus dem Mediumtank 48 oder Spülflüssigkeit aus dem Reservoir 36 zugeführt werden, wobei mit der Zufuhr jeweils gleichzeitig die Rückspülung und die damit einhergehende Membranreinigung erfolgt.

Grundsätzlich besteht bei allen der oben beschriebenen Aufbauten die Möglichkeit, die Spülflüssigkeit nicht (nur) über den permeatseitigen Anschluss 33, sondern stattdessen oder zusätzlich auch über den permeatseitigen Auslass 22, der eigentlich für die Filtratabfuhr vorgesehen ist, in das Filtrationsmodul 16 zu fördern. Hierfür ist dann eine geeignete Verschaltung mit Ventilen vorzusehen, die am permeatseitigen Auslass 22 die Spülflüssigkeitszufuhr von der Filtratabfuhr trennen kann. Im Prinzip kann jeder permeatseitige Anschluss des Filtrationsmoduls 16 (alleine oder in Kombination mit anderen) für die Rückspülung genutzt werden. Obwohl solche Lösungen in den Figuren nicht explizit einzeln dargestellt sind, folgen sie dem gleichen Grundgedanken wie die oben beschriebenen Ausführungsformen.

Bei allen der oben beschriebenen Aufbauten lässt sich die Zellabtrennung aufgrund der Verfügbarkeit der notwendigen Komponenten als vollständig geschlossener Einweg-Prozessschritt abbilden. Die Pumpen 30, 34, 46 (zumindest deren Medium- bzw. Spülflüssigkeit-berührende Teile), die Leitungen 24, 26, 28, 32, 44, 54 sowie die Ventile 38, 40, 42, 50 und die Sensoren sind aus sterilisierbaren Einweg-Materialien gebildet.

Wie ebenfalls bereits oben beschrieben können bei allen Prozessvarianten die Filtrationsleistung permanent gemessen und mit Hilfe einer Steuereinheit die idealen Parameter (insbesondere Zeitpunkt, Dauer, Volumen, Druck, Geschwindigkeit) der Rückspülung ermittelt und entsprechend eingestellt bzw. im Prozess verändert und optimiert werden, um Ausbeute und Effizienz zu optimieren. Hierbei kommen bereits bekannte Einweg-Sensoren zur Messung oder Bestimmung von Druck (insbesondere Transmembrandruck), Fluss, Viskosität, Leifähigkeit sowie deren Veränderungen im Verlauf des Prozesses zum Einsatz.

### Bezugszeichenliste

- 10: Vorrichtungsanordnung
- 12: Vorratsbehälter
- 14: Kulturmedium
- 16: Filtrationsmodul
- 18: Unfiltrat-Einlass
- 20: retentatseitiger Auslass
- 22: permeatseitiger Auslass
- 24: Zulaufleitung
- 26: Rücklaufleitung
- 28: Filtratleitung
- 30: erste Pumpe
- 32: Rückspülleitung
- 33: permeatseitiger Anschluss
- 34: zweite Pumpe
- 36: Reservoir
- 38: erstes Ventil
- 40: zweites Ventil
- 42: drittes Ventil
- 44: Substratzuführleitung
- 46: dritte Pumpe
- 48: Mediumtank
- 50: viertes Ventil
- 52: Sterilfilter
- 54: gemeinsame Zuführleitung

## Patentansprüche

1. Vorrichtungsanordnung (10) zur Abtrennung von Zellen aus einem Kulturmedium in einem Bioprozess, insbesondere in einem biopharmazeutischen Prozess, mit
einem Vorratsbehälter (12) für das Kulturmedium, insbesondere einem Bioreaktor,
einem Filtrationsmodul (16), insbesondere einem Hohlfasermodul, mit wenigstens einer überströmbaren Membran,
einer Zulaufleitung (24), die den Vorratsbehälter (12) mit einem Unfiltrat-Einlass (18) des Filtrationsmoduls (16) verbindet, zum Zuführen von Kulturmedium in das Filtrationsmodul (16),
einer Rücklaufleitung (26), die einen retentatseitigen Auslass (20) des Filtrationsmoduls (16) mit dem Vorratsbehälter (12) verbindet, zum Rückführen von Retentat in den Vorratsbehälter (12),
einer Filtratleitung (28), die an einen permeatseitigen Auslass (22) des Filtrationsmoduls (16) angeschlossen ist, zum Abführen von Filtrat,
einer ersten Pumpe (30) zur Aufrechterhaltung eines Rezirkulationskreislaufs,
einer Rückspülleitung (32), die zu einem permeatseitigen Anschluss (33) und/oder zum permeatseitigen Auslass (22) des Filtrationsmoduls (16) führt, zum Zuführen einer Spülflüssigkeit in das Filtrationsmodul (16), und
einer zweiten Pumpe (34) zum Fördern der Spülflüssigkeit in das Filtrationsmodul (16).

2. Vorrichtungsanordnung (10) nach Anspruch 1, **dadurch gekennzeichnet, dass** die Spülflüssigkeit eines der Folgenden ist: Pufferlösung; Wasser; Substrat.

3. Vorrichtungsanordnung (10) nach Anspruch 1 oder 2, **gekennzeichnet durch** ein erstes Ventil (38) in der Rücklaufleitung (26) zum selektiven Sperren des Retentatrücklaufs in den Vorratsbehälter (12).

4. Vorrichtungsanordnung (10) nach einem der vorhergehenden Ansprüche, **gekennzeichnet durch** ein zweites Ventil (40) in der Filtratleitung (28) zum selektiven Sperren der Abfuhr des Filtrats.

5. Vorrichtungsanordnung (10) nach einem der vorhergehenden Ansprüche, **gekennzeichnet durch** ein drittes Ventil (42) in der Rückspülleitung (32) zum selektiven Sperren der Spülflüssigkeitszufuhr zum Filtrationsmodul (16).

6. Vorrichtungsanordnung (10) nach einem der vorhergehenden Ansprüche, **dadurch gekennzeichnet, dass** die erste Pumpe (30) in die Zulaufleitung (24) integriert ist.

7. Vorrichtungsanordnung (10) nach einem der vorhergehenden Ansprüche, **gekennzeichnet durch** eine Substratzuführleitung (44) zum Zuführen von Substrat in den Vorratsbehälter (12).

8. Vorrichtungsanordnung (10) nach Anspruch 7, **dadurch gekennzeichnet, dass** die Substratzuführleitung (44) direkt in den Vorratsbehälter (12) mündet und dass eine dritte Pumpe (46) zum Fördern des Substrats in den Vorratsbehälter (12) vorgesehen ist.

9. Vorrichtungsanordnung (10) nach Anspruch 7, **dadurch gekennzeichnet, dass** die Substratzuführleitung (44) und die Rückspülleitung (32) stromauf der zweiten Pumpe (34) in eine gemeinsame Zuführleitung (54) münden, die wiederum in den permeatseitigen Anschluss (33) und/oder in den permeatseitigen Auslass (22) des Filtrationsmoduls (16) mündet.

10. Vorrichtungsanordnung (10) nach einem der Ansprüche 7 bis 9, **gekennzeichnet durch** ein viertes Ventil (50) in der Substratzuführleitung (44) zum selektiven Sperren der Substratzufuhr.

11. Vorrichtungsanordnung (10) nach einem der vorhergehenden Ansprüche, **gekennzeichnet durch** einen in die Rückspülleitung (32) oder in die gemeinsame Zuführleitung (54) integrierten Sterilfilter (52).

12. Vorrichtungsanordnung (10) nach einem der vorhergehenden Ansprüche,
**gekennzeichnet durch** Sensoren und eine Steuereinheit zur direkten oder indirekten Bestimmung und Überwachung eines oder mehrerer der folgenden Parameter: Viskosität; elektrische Leitfähigkeit; Fluss, insbesondere Rezirkulationsfluss und/oder Filtratfluss; Druck, insbesondere Transmembrandruck;
wobei die Steuereinheit dazu eingerichtet ist, eine Rückspülung in Abhängigkeit des Erreichens eines oder mehrerer vorbestimmter Grenzwerte der genannten Parameter auszulösen und zu steuern.

13. Verfahren zur Abtrennung von Zellen aus einem Kulturmedium, insbesondere unter Verwendung einer Vorrichtungsanordnung (10) nach einem der vorhergehenden Ansprüche, wobei das Verfahren folgende Schritte aufweist:
- Kultivieren von Zellen in einem Kulturmedium (14) in einem Bioreaktor;
- Filtrieren des Kulturmediums (14) in einem Filtrationsmodul (16) mit wenigstens einer Membran, insbesondere in einem Hohlfasermodul, wobei das Kulturmedium (14) einen Rezirkulationskreislauf durchläuft;
- Spülen der Membran mit einer Spülflüssigkeit während des Filtrierens, wobei die Spülflüssigkeit insbesondere eine Pufferlösung oder Wasser oder Substrat ist, und wobei durch das Spülen Spülflüssigkeit in den Rezirkulationskreislauf eingebracht wird.

14. Verfahren nach Anspruch 13, **dadurch gekennzeichnet, dass** die Spülflüssigkeit über eine separate Leitung (32; 54) in einen oder mehrere permeatseitige(n) Anschluss bzw. Anschlüsse (33, 22) des Filtrationsmoduls (16) gefördert wird.

15. Verfahren nach Anspruch 13 oder 14, **dadurch gekennzeichnet,**
**dass** zumindest während des Filtrierens einer oder mehrerer der folgenden Parameter bestimmt und überwacht werden: Viskosität; elektrische Leitfähigkeit; Fluss, insbesondere Rezirkulationsfluss und/oder Filtratfluss; Druck, insbesondere Transmembrandruck; und
**dass** eine Rückspülung in Abhängigkeit des Erreichens eines oder mehrerer vorbestimmter Grenzwerte der genannten Parameter ausgelöst und gesteuert wird.

16. Verfahren nach einem der Ansprüche 13 bis 15, **dadurch gekennzeichnet, dass** der Schritt des Spülens mehrfach nacheinander durchgeführt wird.

17. Verfahren nach einem der Ansprüche 13 bis 16, **dadurch gekennzeichnet, dass** der Schritt des Spülens während des Filtrierens zyklisch wiederholt wird.

18. Verfahren nach Anspruch 17, **dadurch gekennzeichnet, dass** der wiederholte Schritt des Spülens mit unterschiedlichen Spülflüssigkeiten durchgeführt wird.

19. Verfahren nach einem der Ansprüche 13 bis 18, **dadurch gekennzeichnet, dass** die Schritte des Kultivierens und des Filtrierens nacheinander durchgeführt werden.

20. Verfahren nach einem der Ansprüche 13 bis 18, **dadurch gekennzeichnet, dass** die Schritte des Kultivierens und des Filtrierens zumindest teilweise gleichzeitig durchgeführt werden.

## Claims

1. Device arrangement (10) for separating cells from a culture medium in a bioprocess, in particular in a biopharmaceutical process, comprising
a reservoir (12) for the culture medium, in particular a bioreactor,
a filtration module (16), in particular a hollow fiber module, having at least one membrane across which a flow can take place,
a feed line (24), which connects the reservoir (12) to an unfiltrate inlet (18) of the filtration module (16), for feeding culture medium into the filtration module (16),
a return line (26), which connects a retentate-side outlet (20) of the filtration module (16) to the reservoir (12), for returning retentate to the reservoir (12),
a filtrate line (28), which is connected to a permeate-side outlet (22) of the filtration module (16), for discharging filtrate,
a first pump (30) for maintaining a recirculation circuit,
a back-flushing line (32), which leads to a permeate-side connection (33) and/or to the permeate-side outlet (22) of the filtration module (16), for feeding a flushing liquid into the filtration module (16), and
a second pump (34) for conveying the flushing liquid into the filtration module (16).

2. Device arrangement (10) according to claim 1, **characterized in that** the flushing liquid is one of the following: buffer solution; water; substrate.

3. Device arrangement (10) according to claim 1 or 2, **characterized by** a first valve (38) in the return line (26) for selectively blocking the return of retentate to the reservoir (12) .

4. Device arrangement (10) according to any one of the preceding claims, **characterized by** a second valve (40) in the filtrate line (28) for selectively blocking the discharge of the filtrate.

5. Device arrangement (10) according to any one of the preceding claims, **characterized by** a third valve (42) in the back-flushing line (32) for selectively blocking the feed of flushing liquid to the filtration module (16).

6. Device arrangement (10) according to any one of the preceding claims, **characterized in that** the first pump (30) is integrated in the feed line (24).

7. Device arrangement (10) according to any one of the preceding claims, **characterized by** a substrate feed line (44) for feeding substrate into the reservoir (12).

8. Device arrangement (10) according to claim 7, **characterized in that** the substrate feed line (44) opens directly into the reservoir (12), and **in that** a third pump (46) is provided for conveying the substrate into the reservoir (12).

9. Device arrangement (10) according to claim 7, **characterized in that**, upstream of the second pump (34), the substrate feed line (44) and the back-flushing line (32) open into a joint feed line (54), which in turn opens into the permeate-side connection (33) and/or into the permeate-side outlet (22) of the filtration module (16).

10. Device arrangement (10) according to any one of claims 7 to 9, **characterized by** a fourth valve (50) in the substrate feed line (44) for selectively blocking the feed of substrate.

11. Device arrangement (10) according to any one of the preceding claims, **characterized by** a sterile filter (52) integrated in the back-flushing line (32) or in the joint feed line (54).

12. Device arrangement (10) according to any one of the preceding claims, **characterized by** sensors and a control unit for directly or indirectly determining and monitoring one or more of the following parameters: viscosity; electrical conductivity; flow, in particular recirculation flow and/or filtrate flow; pressure, in particular transmembrane pressure;
wherein the control unit is designed to initiate and control a back-flushing as a function of one or more predetermined limit values of the aforementioned parameters being reached.

13. Method for separating cells from a culture medium, in particular using a device arrangement (10) according to any one of the preceding claims, the method comprising the following steps:
- culturing cells in a culture medium (14) in a bioreactor;
- filtering the culture medium (14) in a filtration module (16) having at least one membrane, in particular in a hollow fiber module, wherein the culture medium (14) passes through a recirculation circuit;
- flushing the membrane with a flushing liquid during the filtration, wherein the flushing liquid is in particular a buffer solution or water or substrate, and wherein the flushing liquid is introduced into the recirculation circuit as a result of the flushing.

14. Method according to claim 13, **characterized in that** the flushing liquid is conveyed via a separate line (32; 54) into one or more permeate-side connection(s) (33, 22) of the filtration module (16).

15. Method according to claim 13 or 14, **characterized**
**in that**, at least during the filtration, one or more of the following parameters is determined and monitored: viscosity; electrical conductivity; flow, in particular recirculation flow and/or filtrate flow; pressure, in particular transmembrane pressure; and
**in that** a back-flushing is initiated and controlled as a function of one or more predetermined limit values of the aforementioned parameters being reached.

16. Method according to any one of claims 13 to 15, **characterized in that** the step of flushing is carried out multiple times one after the other.

17. Method according to any one of claims 13 to 16, **characterized in that** the step of flushing is repeated cyclically during the filtration.

18. Method according to claim 17, **characterized in that** the repeated step of flushing is carried out with different flushing liquids.

19. Method according to any one of claims 13 to 18, **characterized in that** the steps of culturing and filtering are carried out one after the other.

20. Method according to any one of claims 13 to 18, **characterized in that** the steps of culturing and filtering are carried out at least partially simultaneously.

## Revendications

1. Agencement de dispositif (10) pour la séparation de cellules à partir d'un milieu de culture dans un bioprocédé, en particulier dans un procédé biopharmaceutique, avec
un réservoir de stockage (12) pour le milieu de culture, en particulier un bioréacteur,
un module de filtration (16), en particulier un module à fibre creuse, avec au moins une membrane pouvant être submergée,
une conduite d'arrivée (24), qui relie le réservoir de stockage (12) à une entrée de produit à filtrer (18) du module de filtration (16), pour amener le milieu de culture dans le module de filtration (16),
une conduite de retour (26), qui relie une sortie côté rétentat (20) du module de filtration (16) au réservoir de stockage (12), pour renvoyer le rétentat dans le réservoir de stockage (12),
une conduite de filtrat (28), qui est raccordée à une sortie côté perméat (22) du module de filtration (16), pour évacuer le filtrat,
une première pompe (30) pour le maintien d'un circuit de recirculation,
une conduite de rétro-lavage (32), qui mène à un raccord côté perméat (33) et/ou à la sortie côté perméat (22) du module de filtration (16), pour amener un liquide de lavage dans le module de filtration (16), et
une deuxième pompe (34) pour transporter le liquide de lavage dans le module de filtration (16).

2. Agencement de dispositif (10) selon la revendication 1, **caractérisé en ce que** le liquide de lavage est un de ce qui suit : une solution tampon ; de l'eau ; un substrat.

3. Agencement de dispositif (10) selon la revendication 1 ou 2, **caractérisé par** une première soupape (38) dans la conduite de retour (26) pour le blocage sélectif du retour de rétentat dans le réservoir de stockage (12).

4. Agencement de dispositif (10) selon l'une quelconque des revendications précédentes, **caractérisé par** une deuxième soupape (40) dans la conduite de filtrat (28) pour le blocage sélectif de l'évacuation du filtrat.

5. Agencement de dispositif (10) selon l'une quelconque des revendications précédentes, **caractérisé par** une troisième soupape (42) dans la conduite de rétro-lavage (32) pour le blocage sélectif de l'amenée de liquide de lavage vers le module de filtration (16).

6. Agencement de dispositif (10) selon l'une quelconque des revendications précédentes, **caractérisé en ce que** la première pompe (30) est intégrée dans la conduite d'arrivée (24) .

7. Agencement de dispositif (10) selon l'une quelconque des revendications précédentes, **caractérisé par** une conduite d'amenée de substrat (44) pour amener un substrat dans le réservoir de stockage (12).

8. Agencement de dispositif (10) selon la revendication 7, **caractérisé en ce que** la conduite d'amenée de substrat (44) débouche directement dans le réservoir de stockage (12) et qu'une troisième pompe (46) est prévue pour le transport du substrat dans le réservoir de stockage (12).

9. Agencement de dispositif (10) selon la revendication 7, **caractérisé en ce que** la conduite d'amenée de substrat (44) et la conduite de rétro-lavage (32) débouchent en amont de la deuxième pompe (34) dans une conduite d'amenée (54) commune, qui débouche pour sa part dans le raccord côté perméat (33) et/ou dans la sortie côté perméat (22) du module de filtration (16).

10. Agencement de dispositif (10) selon l'une quelconque des revendications 7 à 9, **caractérisé par** une quatrième soupape (50) dans la conduite d'amenée de substrat (44) pour le blocage sélectif de l'amenée de substrat.

11. Agencement de dispositif (10) selon l'une quelconque des revendications précédentes, **caractérisé par** un filtre stérile (52) intégré dans la conduite de rétro-lavage (32) ou dans la conduite d'amenée (54) commune.

12. Agencement de dispositif (10) selon l'une quelconque des revendications précédentes,
**caractérisé par** des capteurs et une unité de commande pour la détermination directe ou indirecte et la surveillance d'un ou plusieurs des paramètres suivants : la viscosité ; la conductivité électrique ; le flux, en particulier le flux de recirculation et/ou le flux de filtrat ; la pression, en particulier la pression transmembranaire ;
dans lequel l'unité de commande est conçue pour déclencher et pour commander un rétro-lavage en fonction de l'atteinte d'une ou plusieurs valeurs limites prédéfinies des paramètres cités.

13. Procédé pour la séparation de cellules à partir d'un milieu de culture, en particulier au moyen d'un agencement de dispositif (10) selon l'une quelconque des revendications précédentes, dans lequel le procédé présente les étapes suivantes :
- la culture de cellules dans un milieu de culture (14) dans un bioréacteur ;
- la filtration du milieu de culture (14) dans un module de filtration (16) avec au moins une membrane, en particulier dans un module à fibre creuse, dans lequel le milieu de culture (14) traverse un circuit de recirculation ;
- le lavage de la membrane avec un liquide de lavage pendant la filtration, dans lequel le liquide de lavage est en particulier une solution tampon ou de l'eau ou un substrat, et dans lequel le liquide de lavage est introduit dans le circuit de recirculation par le lavage.

14. Procédé selon la revendication 13, **caractérisé en ce que** le liquide de lavage est transporté par l'intermédiaire d'une conduite (32 ; 54) séparée dans un ou plusieurs raccord ou raccords côté perméat (33, 22) du module de filtration (16).

15. Procédé selon la revendication 13 ou 14, **caractérisé en ce**
**qu'**au moins pendant la filtration un ou plusieurs des paramètres suivants sont déterminés et surveillés : la viscosité ; la conductivité électrique ; le flux, en particulier le flux de recirculation et/ou le flux de filtrat ; la pression, en particulier la pression transmembranaire ; et
**qu'**un rétro-lavage est déclenché et commandé en fonction de l'atteinte d'une ou plusieurs valeurs limites prédéfinies des paramètres cités.

16. Procédé selon l'une quelconque des revendications 13 à 15, **caractérisé en ce que** l'étape du lavage est mise en œuvre à plusieurs reprises successivement.

17. Procédé selon l'une quelconque des revendications 13 à 16, **caractérisé en ce que** l'étape du lavage est répétée de manière cyclique pendant la filtration.

18. Procédé selon la revendication 17, **caractérisé en ce que** l'étape répétée du lavage est mise en œuvre avec différents liquides de lavage.

19. Procédé selon l'une quelconque des revendications 13 à 18, **caractérisé en ce que** les étapes de la culture et de la filtration sont mises en œuvre successivement.

20. Procédé selon l'une quelconque des revendications 13 à 18, **caractérisé en ce que** les étapes de la culture et de la filtration sont mises en œuvre au moins en partie simultanément.
